# EUROPEAN PATENT APPLICATION

(11) **EP 3 991 746 A1**
(43) Date of publication of application: **04.05.2022**
(21) Application number: 20833398.9
(22) Date of filing: 25.06.2020
(51) Int. Cl.: A61K 39/395, A61K 48/00, A61P 37/00, A61K 9/10, C12N 15/64

(54) **TRANSFECTION METHOD**

(30) Priority: 26.06.2019 JP 2019119164
(71) Applicant: Takeda Pharmaceutical Company Limited, Osaka 541-0045 (JP)
(72) Inventor: MURATA, Naoyuki, Fujisawa-shi, Kanagawa 251-0012 (JP); CHUANOI, Sayan, Fujisawa-shi, Kanagawa 251-0012 (JP); YANAI, Shigeo, Fujisawa-shi, Kanagawa 251-0012 (JP)
(74) Representative: Jones, Nicholas Andrew
(86) International application number: PCT/JP2020/025035
(87) International publication number: WO 2020/262540

(57) **Abstract**

A novel means for safely and efficiently introducing a target substance such as nucleic acid, protein, or the like into immune cells such as T cell and the like is provided by the present invention. Thus, a system for delivering a target substance into an immune cell, including ultrafine bubble water or ultrafine bubble aqueous solution containing ultrafine bubbles with an average diameter of not more than 200 nm and not containing phospholipid, and an ultrasound generator in combination; a method for increasing the delivery of a nucleic acid or a protein into an immune cell by using the ultrafine bubble water, etc. and ultrasound; and the like are provided.

## Description

### [Technical Field]

The present invention relates to a system for delivering a target substance into immune cells, including ultrafine bubble water or an ultrafine bubble aqueous solution containing ultrafine bubbles, and an ultrasonic generator in combination, a method for increasing the delivery of a nucleic acid or a protein into immune cells by using the ultrafine bubble water or aqueous solution, and ultrasound, a preparation containing a nucleic acid or a protein, and the ultrafine bubble water or aqueous solution in combination for delivering the nucleic acid or protein into immune cells by the combined use with ultrasound exposure, and a method for delivering a nucleic acid or a protein into immune cells by contacting the cell with the preparation, and treating the cell with ultrasound, and the like.

### (Background of the Invention)

Ultrasound has been mainly used as an ultrasound imaging apparatus in the medical field. Micro-bubble serving as an ultrasound contrast agent is making an epoch-making progress in ultrasound diagnosis. Recently, it has become possible to use ultrasound for purposes other than diagnosis. For example, non-invasive cancer hyperthermic therapy by focusing ultrasonic energy on the affected part and heating only the affected part is clinically applied for hysteromyoma and prostate cancer. In addition, focusing on noninvasiveness and ease of spatial and temporal control, research is also underway to use ultrasound exposure as a tool for drug delivery system (DDS) that delivers genes and drugs to target cells (sonoporation).

Heretofore, as a gene delivery system for skeletal muscle in which bubble liposome and ultrasound technique are fused, a bubble liposome in which perfluoropropane is encapsulated in a polyethylene glycol(PEG)-modified liposome has been reported (non-patent document 1). In addition, a therapeutic drug for Duchenne muscular dystrophy for administering a PEG-modified bubble liposome (bubble lipopolyplex) with a specific morpholino oligomer bound on the surface, encapsulating perfluorohydrocarbon, and having an average particle size of 50 - 500 nm into the muscle tissue or blood vessel, followed by ultrasound exposure to the muscle tissue from outside the body, to achieve highly efficient introduction of the morpholino oligomer into muscle cells has also been reported (patent document 1). It has also been reported that gas-encapsulated micro-bubble and ultrasound can be used for delivering drugs and genes to the brain (non-patent document 2).

It has been reported that bubble liposomes smaller in size than those using perfluorobutane gas or nitrogen gas are obtained using perfluoropropane gas as a filler gas (non-patent document 3). In addition, it has been reported that administration of bubble lipopolyplex, which is a combination of nano-bubbles and plasmid to ddY mice and exposure of ultrasound to the brain tissue produced the introduction of the plasmid into the vascular endothelium or extravascular region, and the introduction site varies depending on the gas encapsulation efficiency (non-patent document 4).

However, the use of bubble liposome is feared to cause problems of antigenicity due to the lipid. In addition, ultrasound exposure at an output intensity of 1.5 to 2.5 W/cm² which poses concerns for safety of ultrasound diagnosis leads to a problem in terms of practicality.

The present inventors have clarified that nano-bubble water containing nano-bubbles not containing phospholipid at not less than 2.0×10⁸ bubbles/mL has a superior antibacterial action (patent document 2). However, there is no report on the introduction of a target substance such as nucleic acid, protein and the like into cells by combining the nano-bubble water and ultrasound.

The research and development of cancer immunotherapy using CAR-T cells or TCR-T cells transfected with chimeric antigen receptor (CAR) or T cell receptor (TCR) derived from cancer antigen-specific killer T cell is rapidly progressing. The current CAR-T cell therapy generally employs a method in which a CAR gene is introduced ex vivo into T cells, which are collected from a patient, by using a viral vector such as a lentiviral vector, etc. to produce CAR-T cells, and the CAR-T cells are administered to the patient, like Kymriah (trade name) and Yescarta (trade name) approved in the United States. However, this method has a problem that the production cost is high due to the cost of cell culture and preparation of viral vector, and the like. If CAR and exogenous TCR can be selectively introduced into immune cells such as T cell, etc. in vivo, preparation in ex vivo becomes unnecessary, and CAR- or TCR-immune cell therapy with low production cost can be provided. Also, even in ex vivo, if CAR and exogenous TCR can be selectively introduced into immune cells such as T cell, etc. without using a viral vector with a high production cost, the cost for residual virus test and the like becomes unnecessary, and CAR- or TCR-immune cell therapy with low production cost can be provided.

However, since immune cells such as T cell, and the like have a small number of cell divisions and are floating cells, the transfection efficiency is known to be lower compared to other cells. Thus, a more efficient method for introducing nucleic acid, and the like into immune cells is desired. There have been no reports to date on the selective introduction of nucleic acids encoding CAR or exogenous TCR (e.g., mRNA, DNA) into immune cells such as T cell, and the like by using sonoporation.

### [Document List]

### [Patent documents]

patent document 1: WO 2012/153635
patent document 2: WO 2015/182647

### [Non-patent documents]

non-patent document 1: YAKUGAKU ZASSHI 130(11) 1489-1496 (2010)
non-patent document 2: NATURE REVIEWS 12 161-174 (2016)
non-patent document 3: Drug Delivery 24(1) 320-327 (2017)
non-patent document 4: "Evaluation of pharmacokinetics in the brain by ultrasound-responsive nano-bubbles in brain-directed DDS", Yuki Fuchigami et al., Nagasaki University, Abstracts of the 137th Annual Meeting of the Pharmaceutical Society of Japan (March 2017)

### [Summary of Invention]

### [Technical Problem]

An object of the present invention is to provide a novel means for safely and efficiently introducing a target substance such as nucleic acid, protein, or the like into immune cells such as T cell and the like.

### [Solution to Problem]

To achieve the above-mentioned purpose, the present inventors took note of the fact that the nano-bubble water having a superior antibacterial action, which was developed previously by the present inventors (see the above-mentioned patent document 2; since International Organization for Standardization (ISO) defines bubbles smaller than 1 µm (1000 nm) as "ultrafine bubbles" (ISO 20480-1), they are hereinafter referred to as "ultrafine bubbles" instead of "nano-bubbles" in the present specification) does not contain phospholipid. The ultrafine bubble contained in this ultrafine bubble water has an average diameter of not more than 200 nm which is smaller than the conventional diameter. Conventionally, it has been considered that ultrafine bubbles more severely damage cell membrane than microbubbles when crushed. However, the present inventors dared to combine this ultrafine bubble water with ultrasound exposure and investigated the introduction of nucleic acid and protein into T cells. As a result, it was surprisingly shown that the ultrafine bubble water can strikingly increase the introduction efficiency of nucleic acid by sonoporation and, in protein introduction, the combination of ultrafine bubble water and ultrasound can remarkably increase the introduction efficiency than a single treatment with any of them.

Based on these findings, the present inventors have further studied and completed the present invention.

That is, the present invention relates to
[1] a system for delivering a target substance into an immune cell, comprising ultrafine bubble water or ultrafine bubble aqueous solution comprising ultrafine bubbles with an average diameter of not more than 200 nm and not containing phospholipid, and an ultrasound generator in combination;
[2] the system of [1], wherein the ultrafine bubble aqueous solution comprises one or more components selected from one or more kinds of surfactants selected from an anionic surfactant, a non-ionic surfactant, a cationic surfactant and an amphoteric surfactant, a hydrophilic resin, and a buffer;
[3] the system of [1] or [2], wherein the ultrafine bubble aqueous solution consists of a non-ionic surfactant and/or a hydrophilic resin;
   [3-1] the system of [1] or [2], wherein the ultrafine bubble aqueous solution consists of an ionic surfactant and/or a hydrophilic resin;
[4] the system of any of [1] to [3], wherein the ultrafine bubble is constituted of perfluorohydrocarbon or air;
[5] the system of any of [1] to [4], wherein the ultrafine bubble has an average diameter of 50 nm - 200 nm;
[6] the system of any of [1] to [5], wherein the ultrafine bubble has a d90/d10 ratio of not more than 5;
[7] the system of any of [1] to [6], wherein the ultrafine bubble in the ultrafine bubble water or ultrafine bubble aqueous solution has a density of not less than 1.0×10⁸ bubbles/mL;
[8] the system of any of [1] to [7], wherein ultrasound output intensity in the ultrasound generator is not more than 720 mW/cm²;
[9] the system of any of [1] to [8], wherein in the ultrasound generator, ultrasound output intensity is 50 - 500 mW/cm² and ultrasound frequency is 0.5 - 10 MHz;
[10] the system of any of [1] to [9], wherein the target substance is a nucleic acid or a protein;
[11] the system of [10], wherein the nucleic acid encodes a chimeric antigen receptor or an exogenous T cell receptor;
[12] the system of any of [1] to [11], wherein the immune cell is T cell;
[13] the system of [11] or [12], for delivering a nucleic acid into an immune cell;
[14] a method for increasing the delivery of a nucleic acid or a protein into an immune cell by using ultrafine bubble water or ultrafine bubble aqueous solution comprising ultrafine bubble with an average diameter of not more than 200 nm and not containing phospholipid, and ultrasound;
[15] a preparation comprising a combination of a nucleic acid or a protein, and ultrafine bubble water or ultrafine bubble aqueous solution comprising ultrafine bubble with an average diameter of not more than 200 nm and not containing phospholipid, for delivering the nucleic acid or protein into an immune cell, wherein an effective amount of the nucleic acid or protein is delivered into the immune cell by the combined use with ultrasound exposure;
[16] a method for delivering a nucleic acid or a protein into an immune cell by contacting the cell with the preparation of [15], and treating them with ultrasound;
and the like.

### [Advantageous Effects of Invention]

According to the system for delivering a target substance into immune cells of the present invention, the target substances such as nucleic acid, protein, and the like can be non-invasively and selectively introduced into immune cells such as T cells and the like by irradiating ultrasound at an output intensity that does not adversely affect the living body. Therefore, a highly safe introduction system can be provided. In the system, since use of liposome is not required, addition of a special additive such as phospholipid is not necessary, production at a low cost can be realized, and the problem of antigenicity due to phospholipid does not occur.

Conventionally, it has been considered that ultrafine bubbles more severely damage cell membrane than microbubbles when crushed, and thus are not suitable for cell transfection. Surprisingly, according to the present invention, the introduction efficiency of a target substance into a cell can be remarkably increased using the ultrafine bubbles as compared with the case when microbubbles are used.

Furthermore, the ultrafine bubbles having an average diameter of not more than 200 nm are stable for a long term in ultrafine bubble water or ultrafine bubble aqueous solution.

### [Brief Description of Drawings]

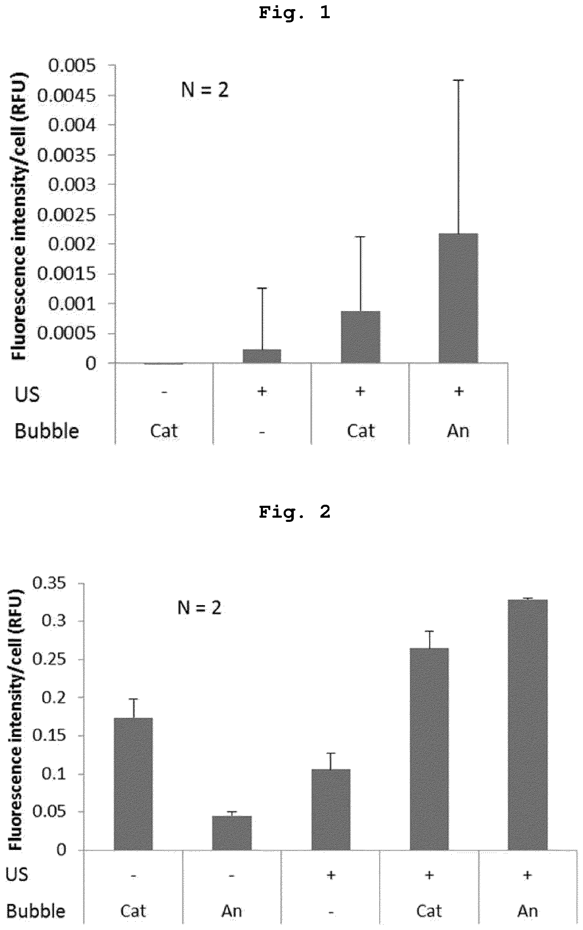

Fig. 1 shows that the introduction efficiency of plasmid into T cells was improved by combining an ultrafine bubble aqueous solution and ultrasound. The vertical axis shows the fluorescence intensity of GFP per surviving cells. US (ultrasound exposure): - (none), + (yes); Bubble (ultrafine bubble): Cat (plus charge), An (minus charge), - (none)
Fig. 2 shows that the introduction efficiency of protein into T cells was improved by combining an ultrafine bubble aqueous solution and ultrasound. The vertical axis shows the fluorescence intensity of GFP per surviving cells. US (ultrasound exposure): - (none), + (yes); Bubble (ultrafine bubble): Cat (plus charge), An (minus charge), An (minus charge), - (none)

### (Detailed Description of the Invention)

### I. The system of the present invention

The present invention provides a system for delivering a target substance into an immune cell, the system containing ultrafine bubble water or ultrafine bubble aqueous solution containing ultrafine bubbles with an average diameter of not more than 200 nm and not containing phospholipid, and an ultrasound generator in combination (hereinafter to be also referred to as "the system of the present invention").

In the present specification, "delivering a target substance into an immune cell" means that a substance that is originally difficult to pass through a cell membrane (for example, a compound that is water-soluble and does not diffuse passively, a compound having a large molecular weight, a compound free of a selective transporter or receptor) passes through a cell membrane and is transferred into a cell. Therefore, the system of the present invention may deliver the target substance into a cell by any mechanism, and examples thereof include, but are not limited to, temporarily opening a pore in the cell membrane.

In the present specification, the "immune cell" means a cell involved in an immune response, and examples include lymphocytes such as T cell, B cell, natural killer (NK) cell, natural killer T (NKT) cell, and the like, granulocytes such as neutrophil, eosinophil, basophil and the like, monocyte, macrophage, dendritic cell, etc., and unipotent or pluripotent stem cells or progenitor cells capable of finally differentiating into them (excluding embryos and other totipotent or pluripotent stem cells). The "immune cell" may be an isolated specific immune cell or a non-uniform cell population including multiple types of immune cells, such as lymphocytes, as long as it is a cell population containing any of the above-mentioned cells.

The aforementioned lymphocyte can be produced by collecting from, for example, peripheral blood, bone marrow and cord blood of humans or non-human mammals, or differentiating from stem cells such as iPS cell, and the like by a method known per se. When isolated immune cells (e.g., T cells) into which the target substance has been introduced by applying the present invention are used for the treatment of diseases such as cancer and the like, the cells are preferably collected from a subject to be treated itself or a donor matched to the MHC type of the subject to be treated. Alternatively, the lymphocyte may be one present in an individual animal.

In one preferable embodiment, the "immune cell" is an isolated and purified T cell. In another preferred embodiment, the immune cell may be a cell population containing T cells, for example, T cell and the like present in an individual animal.

In the present specification, the "T cell" means a type of leukocyte found in lymphatic organs or peripheral blood, etc., and a classification of lymphocytes characterized by differentiation and maturation mainly in the thymus and expression of TCR. Examples of the T cell that can be used in various embodiments of the present invention include cytotoxic T cell (CTL) which is a CD8-positive cell, helper T cell, regulatory T cell, effector T cell which are CD4-positive cells, and the like, and cytotoxic T cell is preferred.

The target substance to be delivered into an immune cell by the system of the present invention is not particularly limited as long as it is a substance capable of imparting preferable physiological activity to the immune cell as a result of the delivery into the cell. Examples thereof include, but are not limited to, polymer compounds [for example, nucleic acid, small RNA/DNA such as siRNA (e.g., FAM-siRNA, manufactured by NIPPON GENE CO., LTD.), ssRNA, shRNA, miRNA, S-oligo DNA (phosphorothioate), etc.), genes (e.g., plasmid DNA, mRNA, etc.), and the like, proteins (also including peptides) (e.g., antibody (e.g., IgG (e.g., Alexa-IgG, manufactured by Invitrogen)), etc.), polysaccharides (e.g., dextran, fluorescein isothiocyanate dextran, etc.) and the like], low-molecular-weight compounds (e.g., fluorescein, fluorescein sodium, etc.) and the like. Among these, polymer compounds and low-molecular-weight compounds can be preferably mentioned. They are further preferably polymer compounds. They are further preferably nucleic acid and proteins. They are particularly preferably nucleic acids and antibodies.

In the present specification, the "ultrafine bubble" may contain a gas having a general atmospheric pressure or a pressure higher than that, and the inside of the ultrafine bubble may be a vacuum. As used herein, "vacuum" means a state of a space filled with a gas having a pressure lower than the normal atmospheric pressure.

In the present invention, the "ultrafine bubble not containing phospholipid" refers to an ultrafine bubble in which the shell of the bubble does not form the structure of phospholipid bilayer.

In the present invention, the "ultrafine bubble water" refers to water containing ultrafine bubbles.

In the present invention, the "ultrafine bubble aqueous solution" refers to an aqueous solution containing ultrafine bubbles. The aqueous solution constituting the ultrafine bubble aqueous solution contains, for example, one or more kinds of substances selected from
1) one or more kinds of surfactants selected from an anionic surfactant, a non-ionic surfactant, a cationic surfactant and an amphoteric surfactant,
2) a hydrophilic resin and
3) a buffer
as components.

Examples of the "anionic surfactant" in the present invention include sodium lauryl sulfate and the like.

Examples of the "non-ionic surfactant" in the present invention include glycerol fatty acid ester (e.g., glycerol monostearate, etc.), sucrose fatty acid ester, sorbitan fatty acid ester (e.g., sorbitan monostearate, sorbitan monolaurate, etc.), polyglycerin fatty acid ester, polyoxyethylene (hydrogenated) castor oil, polyoxyethylene sorbitan fatty acid ester (e.g., sorbitan polyoxyethylene lauric acid ester (e.g., polysorbate 20, etc.), polyoxyethylene sorbitan oleic acid ester (e.g., polysorbate 80, etc.), etc.), polyethylene glycol fatty acid ester, polyoxyethylene alkyl ether (e.g., polyoxyethylene lauryl ether, etc.), polyoxyethylene polyoxypropylene alkyl ether (e.g., polyoxyethylene polyoxypropylene cetyl ether, etc.), polyoxyethylene alkylphenyl ether (e.g., polyoxyethylene nonylphenyl ether, etc.), macrogols, polyoxyethylene polyoxypropylene glycol (e.g., poloxamer 407, poloxamer 235, poloxamer 188, poloxamine, etc.) and the like. Among these, polyoxyethylene sorbitan lauric acid ester (e.g., polysorbate 20, etc.), polyoxyethylene sorbitan oleic acid ester (e.g., polysorbate 80, etc.) are preferable. Polysorbate 20 or polysorbate 80 is further preferable, and polysorbate 80 is particularly preferable.

Examples of the "cationic surfactant" in the present invention include benzalkonium chloride, benzethonium chloride, cetylpyridinium chloride, hexadecyltrimethylammonium bromide, dequalinium chloride and the like.

Examples of the "amphoteric surfactant" in the present invention include cocamidepropyl betaine, cocamidepropyl hydroxysultaine and the like.

The above-mentioned surfactants may be used alone, or two or more kinds thereof may be used in combination.

Examples of the "hydrophilic resin" in the present invention include acrylic resin (e.g., polyacrylamide, polyacryl acid, polymethyl methacrylate), vinyl resin (e.g., polyvinylpyrrolidone, poly(vinyl alcohol) (PVA), polyvinyl ethyl ether); polysaccharide (e.g., tragacanth gum, caraya gum, methylcellulose, hydroxypropylcellulose, hydroxypropylmethylcellulose, hyaluronic acid, agarose, curdlan etc.). Among these, poly(vinyl alcohol), hydroxypropylcellulose are preferable. It is more preferably poly(vinyl alcohol).

The above-mentioned hydrophilic resins alone, or two or more kinds thereof may be used in combination.

Examples of the "buffer" in the present invention include acidic buffer (e.g., acetate buffer, citrate buffer, diluted Mclivaine buffer), neutral buffer (e.g., 4-(2-hydroxyethyl)-1-piperazineethanesulfonic acid (HEPES) buffer, tris (hydroxymethyl)aminomethane (Tris) buffer, phosphate buffer, and phosphate buffered saline (PBS)). As the buffer, diluted Mclivaine buffer is preferred.

As an aqueous solution which constitutes the "ultrafine bubble aqueous solution" in the present invention is preferably an aqueous solution or the like composed of 1) one or more kinds of surfactants selected from an anionic surfactant, a non-ionic surfactant, a cationic surfactant and an amphoteric surfactant, and/or 2) hydrophilic resin. Among these, for example, an aqueous solution and the like composed of a non-ionic surfactant and/or a hydrophilic resin are preferable. In addition, an aqueous solution and the like composed of a non-ionic surfactant are also preferable. An aqueous solution or the like composed of 1) one or two kinds selected from polysorbate 80 and polysorbate 20 and/or 2) poly(vinyl alcohol) is further preferable. An aqueous solution or the like composed of polysorbate 80 and/or poly(vinyl alcohol) is further preferable. An aqueous solution composed of polysorbate 80 is particularly preferable.

In another preferred embodiment, as an aqueous solution which constitutes the "ultrafine bubble aqueous solution", for example, an aqueous solution composed of an ionic (anionic or cationic) surfactant and/or a hydrophilic resin can be mentioned.

Examples of the "gas" which constitutes ultrafine bubble in the present invention include, but are not limited to, one kind or a mixture of two or more kinds selected from perfluorohydrocarbon (e.g., perfluoropropane (C₃F₈), perfluorobutane, etc.), air, nitrogen, ozone, oxygen, argon, carbon dioxide and helium and the like. Among these, perfluorohydrocarbon (e.g., perfluoropropane, perfluorobutane, etc.), air, nitrogen, ozone, oxygen, argon are preferable. Perfluorohydrocarbon (e.g., perfluoropropane, perfluorobutane, etc.), air are more preferable. When air is used, ultrafine bubbles can be produced easily at a low cost. Further preferred is air.

The "ultrafine bubble aqueous solution" in the present invention is preferably an ultrafine bubble aqueous solution composed of (A) an aqueous solution composed of a non-ionic surfactant and/or a hydrophilic resin and (B) an ultrafine bubble constituted of one or more kinds of gas selected from perfluorohydrocarbon, air and the like. An ultrafine bubble aqueous solution composed of (A) an aqueous solution composed of 1) one or two kinds selected from polysorbate 80 and polysorbate 20 and/or 2) poly(vinyl alcohol) and (B) an ultrafine bubble constituted of one or two kinds of gas selected from perfluorohydrocarbon and air is further preferable. An ultrafine bubble aqueous solution composed of (A) an aqueous solution composed of polysorbate 80 and/or poly(vinyl alcohol) and (B) an ultrafine bubble constituted of perfluorohydrocarbon and/or air are further preferable. An ultrafine bubble aqueous solution composed of polysorbate 80 and an ultrafine bubble constituted of air is particularly preferable.

The "ultrafine bubble" in the present invention does not containing amphiphilic phospholipids such as liposome and the like. Accordingly, a safer preparation that does not show antigenicity can be provided.

The "ultrafine bubble" in the present invention has an average diameter of about not more than 200 nm. The average diameter is preferably 10 nm - 200 nm, further preferably 50 nm - 200 nm, more preferably 100 nm - 180 nm.

The "average diameter" in the present specification means the particle size (mode diameter) corresponding to the most frequent value of distribution (maximum value of number %).

In the present specification, the "ultrafine bubble water" or "ultrafine bubble aqueous solution" means water or aqueous solution in which gas particles (ultrafine bubbles) having a diameter of not more than 1000 nm are stably present. The ultrafine bubble water or ultrafine bubble aqueous solution in the present invention (hereinafter to be also referred to as "ultrafine bubble water, etc. in the present invention") characteristically contains ultrafine bubbles with an average diameter of not more than about 200 nm.

In the present invention, the ultrafine bubbles desirably have a uniform size. For example, when the ultrafine bubble diameters corresponding to cumulative 10% and cumulative 90% from the smaller diameter side of the ultrafine bubble number-based distribution are d10 and d90, respectively, the "d90/d10 ratio" is preferably not more than 5, further preferably not more than 4.5.

In the present invention, the number of ultrafine bubbles contained in the ultrafine bubble water, etc. means the number of ultrafine bubbles present in 1 mL of the ultrafine bubble water or ultrafine bubble aqueous solution, which is sometimes to be referred to as "ultrafine bubble density" in the present specification. The number of ultrafine bubbles contained in the ultrafine bubble water, etc. in the present invention is not particularly limited. The lower limit of the "ultrafine bubble density" is, for example, not less than 1.0×10⁸ bubbles/mL, preferably not less than 2.0×10⁸ bubbles/mL, more preferably not less than 2.5×10⁸ bubbles/mL. The upper limit of the "ultrafine bubble density" is, for example, not more than 2.0×10⁹ bubbles/mL, preferably not more than 1.0×10⁹ bubbles/mL. The "ultrafine bubble density" in the present invention is, for example, 1.0×10⁸ - 2.0×10⁹ bubbles/mL, preferably 2.0×10⁸ - 1.0×10⁹ bubbles/mL, further preferably 2.5×10⁸ - 1.0×10⁹ bubbles/mL.

The ultrafine bubble diameter (including ultrafine bubble average diameter, hereinafter the same), ultrafine bubble number-based distribution (including d90/d10 ratio, hereinafter the same), and ultrafine bubble number can be measured by a method using scattering of laser beam based on Brownian motion (e.g., NanoSight Ltd, LM20, LM10, etc.), a method based on electric resistance change (e.g., Beckman Coulter, Multisizer4, etc.), a method based on laser diffraction scattering method (e.g., Shimadzu Corporation, SALD-7100H, etc.), a method using Mie scattering (e.g., NIPPON DENSHOKU INDUSTRIES CO., LTD., NP-500T, etc.) and the like. The ultrafine bubble diameter and ultrafine bubble number-based distribution used in the present invention are those measured by a tracking method (tracking method) using laser beam scattering using NanoSight (instrument name LM10) manufactured by NanoSight Ltd., or in accordance therewith.

The ultrafine bubble diameter, ultrafine bubble number-based distribution and ultrafine bubble number may be generally measured immediately after production of ultrafine bubble water, etc., or measured after long-term storage. The ultrafine bubble diameter, ultrafine bubble number-based distribution and ultrafine bubble number of the ultrafine bubble water, etc. in the present invention are stably maintained for an extremely long time (e.g., about 6 months to 2 years), and thus, the ultrafine bubble diameter, ultrafine bubble number-based distribution and ultrafine bubble number may be measured immediately before use after sealed storage for a certain period after production of the ultrafine bubble water.

In the present specification, the "water" containing ultrafine bubbles (and "water" used as an "aqueous solution" containing ultrafine bubbles) is not particularly limited and, for example, tap water, deionized water, distilled water, sterile distilled water, purified water for injection, ultrapure water and the like can be used. For use for injection, sterile distilled water, purified water for injection and the like are preferable.

Examples of the "aqueous solution" containing ultrafine bubbles in the present specification include water containing one or more of the aforementioned components selected from one or more kinds of surfactants selected from an anionic surfactant, a non-ionic surfactant, a cationic surfactant and an amphoteric surfactant, hydrophilic resin, and buffering agent, and further containing, for example, any additive generally used in the field of pharmaceutical preparation. Examples of the "additive" include electrolyte, excipient, lubricant, binder, disintegrant, solubilizing agent, suspending agent, dispersing agent, isotonicity agent, soothing agent, antiseptic, antioxidant, colorant, sweetening agent, pH adjuster, stabilizer, acidulant, flavor, fluidizer and the like. Pharmacologically acceptable additives are preferable. One or more kinds of additives selected from suspending agent, stabilizer, dispersing agent, isotonicity agent and the like are more preferably used.

Two or more kinds of the above-mentioned additives may be used in a mixture at appropriate ratios.

These additives (including one or more kinds of surfactants selected from an anionic surfactant, a non-ionic surfactant, a cationic surfactant and an amphoteric surfactant, hydrophilic resin, and buffering agent) can also be directly dissolved in water to prepare a ultrafine bubble aqueous solution as long as they do not affect the generation, stability and the like of the ultrafine bubbles, or the ultrafine bubbles are generated in water free of additive to give ultrafine bubble water and additives are dissolved when in use to give an ultrafine bubble aqueous solution.

As the aqueous solution, any of an uncharged ultrafine bubble aqueous solution, a positively-charged ultrafine bubble aqueous solution and a negatively-charged ultrafine bubble aqueous solution can be used. They can be properly selected according to the kind of the target immune cell, their surrounding microenvironment, or the kind of the target substance, or the like. In the method of the present invention for delivery of the target substance into an immune cell by using the ultrafine bubble aqueous solution and ultrasound, for example, when the target substance is a polymer compound (e.g., nucleic acid, protein, etc.), the ultrafine bubble aqueous solution is preferably a negatively-charged ultrafine bubble aqueous solution in some cases. In another embodiment, in the method of the present invention for delivery of the target substance into an immune cell by using the ultrafine bubble aqueous solution and ultrasound, when the target substance is a polymer compound, the ultrafine bubble aqueous solution is preferably a positively-charged ultrafine bubble aqueous solution in some cases.

The charge of the ultrafine bubble aqueous solution can be appropriately adjusted by, for example, the pH of the buffer to be used. For example, to make an ultrafine bubble aqueous solution positively-charged, the pH of the ultrafine bubble aqueous solution is preferably 1 - 4. On the other hand, to make an ultrafine bubble aqueous solution negatively-charged, the pH of the ultrafine bubble aqueous solution is preferably 7 - 14.

The production methods of ultrafine bubble water are roughly divided into a method including simultaneously generating micro-bubbles (gas particles having a diameter of about 1 - 60 µm) and ultrafine bubbles in water, and floatseparating the micro-bubbles to leave only the ultrafine bubbles, and a method including directly generating ultrafine bubbles, and the former is the mainstream at present. The former method includes a high-speed swirling flow type in which a gas is crushed by high-speed swirling to generate a large number of micro-bubbles, and the micro-bubbles are float-separated to leave ultrafine bubbles in water, a pressurized dissolution type in which a gas is pressurized to be dissolved at supersaturation, the solution is rapidly decompressed to generate micro-bubbles and ultrafine bubbles, the micro-bubbles are float-separated to leave ultrafine bubbles in water, and the like. The production methods of ultrafine bubble aqueous solution are as the same as mentioned above.

The pressurized dissolution type is preferably used as the method for producing ultrafine bubble water or ultrafine bubble aqueous solution in the present invention. For example, the following steps 1) to 3) can be mentioned; 1) in a pressurized container pressurized to about 0.2 to 0.5 MPa by a pressurization pump, a gas is forcibly dissolved in a liquid in which (i) one or more kinds of surfactants selected from an anionic surfactant, a non-ionic surfactant, a cationic surfactant and an amphoteric surfactant, (ii) a hydrophilic resin and/or (iii) buffer solution; 2) flash operation in water through a nozzle is performed to release the depressurized and supersaturated gas into the waste water as micro-bubbles or ultrafine bubbles, whereby a mixture of micro-bubble water and ultrafine bubble water is produced; 3) aeration is discontinued and the mixture is left standing to allow micro-bubbles to be naturally separated by floating. As a result, clear ultrafine bubble water with only ultrafine bubbles remaining therein is produced.

Examples of the ultrafine bubble generator used in producing ultrafine bubble water or ultrafine bubble aqueous solution in the present invention include pressurized dissolution type apparatus (e.g., nanoGALF^{™} manufactured by IDEC, OM4-MD5-045 manufactured by AURA TEC CO., LTD., micro-bubble generator manufactured by Nikuni Corporation, etc.), high-speed swirling flow type apparatus (e.g., YJ manufactured by Bi-clean, micro-bubble generator manufactured by AQUA AIR, MICROBLADE manufactured by ROYAL ELECTRIC CO., LTD., etc.) and the like. As the ultrafine bubble generator, a pressurized dissolution type apparatus (e.g., nanoGALF^{™} manufactured by IDEC) is preferable.

In the present invention, one or more kinds of surfactants selected from an anionic surfactant, a non-ionic surfactant, a cationic surfactant and an amphoteric surfactant, hydrophilic resin and/or buffer solution are used for the production of ultrafine bubble water or ultrafine bubble aqueous solution, whereby the number of ultrafine bubbles in the above-mentioned ultrafine bubble water or ultrafine bubble aqueous solution can be increased.

The content of one or more kinds of surfactants selected from an anionic surfactant, a non-ionic surfactant, a cationic surfactant and an amphoteric surfactant, hydrophilic resin and/or buffer solution used in the present invention in water is not particularly limited. The upper limit is preferably not more than 50% (W/V), more preferably not more than 20% (W/V), further preferably not more than 10% (W/V). The lower limit is preferably not less than 0.01% (W/V), more preferably not less than 0.05% (W/V), further preferably not less than 0.1%(W/V). As used herein, (W/V) means g/mL.

When two or more kinds of the surfactant, hydrophilic resin and buffer solution are used in combination, the total amount thereof is the content in water.

The ultrafine bubble water, etc. in the present invention which is produced as mentioned above is tightly sealed in a vial or ampoule and preserved. The preservation is preferably performed under shading conditions. The preservation temperature is preferably not more than room temperature, and not more than 10°C is more preferable.

The ultrafine bubble water or ultrafine bubble aqueous solution in the present invention is preferably produced in the presence of one or more kinds of surfactants selected from an anionic surfactant, a non-ionic surfactant, a cationic surfactant and an amphoteric surfactant, hydrophilic resin and/or buffer solution. Therefore, the number of ultrafine bubbles in ultrafine bubble water, etc. can be maintained at not less than 1.0×10⁸ bubbles/mL for a period when maintenance of the action effect of the ultrafine bubble water, etc. in the present invention (e.g., cell membraneperforating effect on immune cell when ultrafine bubble water, etc. and ultrasonic treatment are used in combination, effect of increasing the delivery of an the target substance into an immune cell by using ultrafine bubble water, etc. and ultrasound, etc.) is required (e.g., when used as a base for an the target substance that is desired to be delivered through cell membrane, etc., the effective period thereof (e.g., not less than 3 months, more preferably not less than 6 months, further preferably not less than one year)).

The ultrafine bubble water, etc. in the present invention can be sterilized by heating, and the number of ultrafine bubbles can be maintained at not less than 1.0×10⁸ bubbles/mL even after heat sterilization.

When the number of ultrafine bubbles in the ultrafine bubble water, etc. in the present invention is not less than 1.0×10⁸ bubbles/mL, a superior antibacterial action and a preservation effect resulting therefrom are exhibited. Therefore, they are also useful as a base for a liquid pharmaceutical preparation of a multiple administration type which is repeatedly administered from the same container for a certain period of time, for example, injection (e.g., subcutaneous injection, intravenous injection, intramuscular injection, intraperitoneal injection, drip transfusion, intracerebral injection, intra-cerebrospinal fluid injection, intraocular injection, etc.).

The ultrafine bubble water, etc. in the present invention is preferably produced in the presence of one or more kinds of surfactants selected from an anionic surfactant, a non-ionic surfactant, a cationic surfactant and an amphoteric surfactant (preferably polysorbate 80 and/or polysorbate 20, further preferably polysorbate 80), and the ultrafine bubbles in the produced ultrafine bubble water, etc. have a smaller average diameter and more uniform size (i.e., d90/d10 ratio is small). Therefore, a safer effect of delivering the target substance into an immune cell is obtained in the system of the present invention.

As an "ultrasound generator" used in the system of the present invention, any can be used as long as it can generate ultrasonic wave meeting conditions sufficient to deliver a target substance into an immune cell when used in combination with the ultrafine bubble water, etc. in the present invention. For example, any apparatus conventionally used for ultrasound diagnosis in the clinical situation, a commercially available ultrasound gene transfer apparatus (e.g., Sonitron GTS (manufactured by Nepa Gene Co., Ltd.), etc.) and the like can be appropriately used.

The conditions of the "ultrasound generator" in the present invention include, for example, an ultrasound output intensity (acoustic energy passing through a unit area (cm²) perpendicular to the traveling direction of sound waves in a unit time) of not less than 10 mW, preferably not less than 30 mW, more preferably not less than 50 mW. The upper limit of the output intensity is not particularly set. In particular, when the system of the present invention aims at in vivo transfection to an immune cell in mammals including human, a range that does not adversely affect the animals (e.g., cytotoxicity) is preferable as the upper limit. For example, the requirement of "not exceeding 720 mW/cm²" (same as the upper limit of Track 3 of US FDA) was added to the third party certification criteria (2005) of the revised Pharmaceutical Affairs Law, and ultrasound diagnosis apparatuses in Japan are controlled so as not to exceed the upper limit. Thus, the upper limit of the ultrasound output intensity in the system of the present invention is preferably 720 mW/cm². The ultrasound output intensity is preferably 50 - 720 mW/cm², further preferably 50 - 500 mW/cm². The ultrasound output intensity used for conventional gene transfer is significantly higher than the above-mentioned criteria for ultrasound diagnosis applications (e.g., 1.5 - 2.5W/cm²), thus posing a high safety risk. In the system of the present invention, a target substance can be efficiently delivered into an immune cell with a small output intensity (preferably 50 - 500 mW/cm²) regardless of the molecular weight of the target substance, and it is an extremely safe deliver system.

As the conditions of the "ultrasound generator" in the present invention, the ultrasound frequency is not particularly limited and can be appropriately selected, for example, within the range of 0.5 - 10 MHz. The frequency that is widely adopted at present is about 1 MHz. However, since higher frequencies are considered to have less adverse influence on the body, the frequency can be appropriately selected within the range of 1 to 5 MHz, more preferably 1 to 3 MHz.

As the conditions for using the "ultrasound generator" in the present invention, the ultrasound exposure time is not particularly limited as long as it is sufficient to deliver a target substance into an immune cell, and varies depending on the ultrasound output intensity. For example, even when the output intensity is 50 mW/cm², the delivery of a target substance into an immune cell can be achieved by an exposure time of 10 seconds. The ultrasound exposure time may be, for example, 1 to 60 seconds, preferably 1 to 30 seconds, more preferably 1 to 20 seconds.

As the conditions for using the "ultrasound generator" in the present invention, for example, a combination of (i) ultrasound output intensity of 50 to 720 mW/cm² (preferably 50 to 500 mW/cm²), (ii) ultrasound frequency of 0.5 to 10 MHz, and (iii) ultrasound exposure time of 1 to 60 seconds can be mentioned. Among these, a combination of (i) ultrasound output intensity of 50 to 500 mW/cm², (ii) ultrasound frequency of 1 to 5 MHz, and (iii) ultrasound exposure time of 1 to 60 seconds, and the like are preferable.

When the system of the present invention is used for the transfection of an immune cell isolated from an animal, or an immune cell obtained by inducing differentiation of a stem cell such as iPS cell or the like by a method known per se, it may include a step of culturing the immune cells after a transfection treatment. Therefore, the system of the present invention may further include a means for culturing immune cells (e.g., culture container (e.g., dish, flask, etc.), medium, culture apparatus (e.g., CO₂ incubator, etc.)). Since immune cells proliferate in a floating state, a culture container coated with a non-adhesive or low-adhesive substrate is preferred. The above-mentioned culture step also includes a passage culture step. When cells are passage cultured by the system of the present invention, the expression intensity of the target substance introduced into the cell can be maintained.

### II. The delivery-increasing method of the present invention

The present invention also provides a method for increasing the delivery of a nucleic acid or a protein into an immune cell, by using ultrafine bubble water or ultrafine bubble aqueous solution containing ultrafine bubbles with an average diameter of not more than 200 nm and not containing phospholipid, and ultrasound (hereinafter to be also referred to as "the delivery-increasing method of the present invention"). The method includes administering ultrafine bubble water, etc. in the present invention to a subject to deliver same to the vicinity of the immune cell, and performing ultrasound exposure to the immune cell, whereby the nucleic acid or protein is delivered into the immune cell. As used herein, the "immune cell" means the same as that defined for the above-mentioned system of the present invention.

As the ultrafine bubble water or ultrafine bubble aqueous solution to be used in "the delivery-increasing method of the present invention", the above-mentioned ultrafine bubble water, etc. in the present invention can be used. The ultrasound exposure in "the delivery-increasing method of the present invention" can be performed under conditions similar to those for the use of the ultrasound generator in the above-mentioned system of the present invention.

The nucleic acid or protein whose delivery into an immune cell is increased by the delivery-increasing method of the present invention is not particularly limited, and may be, for example, a substance capable of imparting preferable physiological activity to an immune cell as a result of the delivery into the immune cell. Examples of the nucleic acid include, but are not limited to, small RNA/DNA such as siRNA (e.g., FAM-siRNA, manufactured by NIPPON GENE CO., LTD.), ssRNA, shRNA, miRNA, S-oligo DNA (phosphorothioate, etc.), genes (e.g., plasmid DNA, mRNA, etc.), and the like. Examples of the protein include antibody (e.g., IgG (e.g., Alexa-IgG, manufactured by Invitrogen Corp.), etc.), peptide and the like. Among these, a nucleic acid encoding chimeric antigen receptor (CAR) or exogenous T cell receptor (TCR), and the like are preferred as the nucleic acid, and antibody and the like are preferred as the protein.

The number of nucleotides in the nucleic acid and the molecular weight of the protein are not particularly limited. For example, when the nucleic acid is a small RNA/DNA such as siRNA, antisense oligo nucleic acid, or the like, it is 10 mer - 30 mer, preferably 15 mer - 25 mer, and when the nucleic acid is a nucleic acid encoding CAR or TCR (plasmid DNA), it is 1 - 10 kb, preferably 2 - 8 kb. In the case of protein, it is 25 kDa - 900 kDa, preferably 25 kDa - 320 kDa.

In one preferable embodiment, the nucleic acid is a nucleic acid encoding CAR or exogenous TCR. The nucleic acid encoding CAR or exogenous TCR is described in detail in the following.

### (a) Nucleic acid encoding CAR

CAR is an artificially constructed hybrid protein containing an antigen binding domain (e.g., scFv) of an antibody linked to a T cell signal transduction domain. The characteristic of CAR is the ability to convert specificity and reactivity of T cell to the selected target in a non-MHC-restricting manner, by using its antigen-binding property of monoclonal antibody. Non-MHC-restricting antigen recognition imparts the ability to recognize antigen independently of antigen processing to T cells expressing CAR, thereby bypassing the major mechanism of tumor escape. Moreover, when expressed in T cells, CAR advantageously does not dimerize with endogenous TCR α chain and β chain.

CAR used in the present invention includes antigen binding domain of an antibody that can specifically recognize surface antigens (e.g., cancer antigen peptide, surface receptor that is upregulated in cancer cells, etc.) to be recognized by the target immune cell (e.g., T cell, NK cell, NKT cell, monocyte, macrophage, dendritic cell etc.), extracellular hinge domain, transmembrane domain, and intracellular T cell signal transduction domain.

Examples of the surface antigen specifically recognized by the antigen binding domain include, but are not limited to, surface receptors upregulated in various carcinomas (e.g., acute lymphocytic cancer, alveolar rhabdomyosarcoma, bladder cancer, bone cancer, brain cancer (e.g., medulloblastoma), breast cancer, anus, anal canal or anus rectal cancer, eye cancer, intrahepatic bile duct cancer, articular cancer, neck, gall bladder or pleural cancer, nasal, nasal cavity or middle ear cancer, oral cancer, vulvar cancer, chronic myelogenous cancer, colorectal cancer, esophageal cancer, cervical cancer, fibrosarcoma, gastrointestinal carcinoid tumor, head and neck cancer (e.g., head and neck squamous cell carcinoma), hypopharyngeal cancer, kidney cancer, laryngeal cancer, leukemia (e.g., acute lymphoblastic leukemia, acute lymphocytic leukemia, chronic lymphocytic leukemia, acute myelogenous leukemia), liquid tumor, liver cancer, lung cancer (e.g., non-small cell lung cancer), lymphoma (e.g., Hodgkin lymphoma, non-Hodgkin's lymphoma, diffuse large B cell lymphoma, follicular lymphoma), malignant mesothelioma, mastocytoma, melanoma, multiple myeloma, nasopharynx cancer, ovarian cancer, pancreatic cancer; peritoneal, retinal and mesenteric cancer; pharyngeal cancer, prostate cancer, rectal cancer, kidney cancer, skin cancer, small intestinal cancer, soft tissue cancer, solid tumor, gastric cancer, testicular tumor, thyroid cancer, ureteral cancer and the like), for example, CD19, EGF receptor, BCMA, CD30, Her2, ROR1, MUC16, CD20, mesothelin, B-cell mutation antiten (BCMA), CD123, CD3, prostate specific membrane antigen (PSMA), CD33, MUC-1, CD138, CD22, GD2, PD-L1, CEA, chondroitin sulfate proteoglycan-4, IL-13 receptor α chain, IgGK light chain and the like, cancer antigen peptide (e.g., peptide derived from WT1, GPC3, MART-1, gp100, NY-ESO-1, MAGE-A4, or the like), and the like.

The antigen binding domain to be used in the present invention is not particularly limited as long as it is an antibody fragment that can specifically recognize the target antigen. In consideration of the ease of CAR production, it is desirable to use a single-chain antibody (scFv) in which a light chain variable region and a heavy chain variable region are linked via a linker peptide. The arrangement of the light chain variable region and the heavy chain variable region in a single-chain antibody is not particularly limited as long as both can reconstitute a functional antigen-binding domain. They can be designed in the order of light chain variable region - linker peptide - heavy chain variable region from the N-terminal side. As the linker peptide, a linker peptide known per se and generally used for producing a single-chain antibody can be used. DNA encoding a light chain variable region and DNA encoding a heavy chain variable region can be prepared, for example, by cloning light chain gene and heavy chain gene from antibody-producing cells, and performing PCR by using them as templates, or chemically synthesizing them from the sequence information of existing antibodies. The DNA encoding the single-chain antibody can be obtained by ligating each of the obtained DNA fragments and the DNA encoding the linker peptide by an appropriate method. It is preferable that a leader sequence is further added to the N-terminal side of the antigen-binding domain in order to present CAR on the surface of immune cells.

As the extracellular hinge domain and the transmembrane domain, the domains derived from T cell surface molecules generally used in the art can be appropriately used, and examples thereof include, but are not limited to, respective domains derived from CD8a and CD28.

Examples of the intracellular signal transduction domain include, but are not limited to, those having a CD3ζ chain, those further having co-stimulation transducing motif such as CD28, CD134, CD137, Lck, DAP10, ICOS, 4-1BB and the like between transmembrane domain and CD3ζ chain, those having two or more co-stimulation transducing motifs, and the like. Any domains generally used in the art can be used in combination.

The information of the nucleic acid sequence encoding extracellular hinge domain, transmembrane domain and intracellular signal transduction domain is well known in the art, and those of ordinary skill in the art can acquire a DNA fragment encoding respective domains with ease from T cells based on the information.

DNA encoding CAR can be obtained by linking the thus-obtained DNA fragments respectively encoding antigen binding domain, extracellular hinge domain, transmembrane domain and intracellular signal transduction domain.

The thus-obtained DNA encoding CAR can be inserted into an expression vector, preferably a plasmid vector, containing a promoter that is functional in T cells, directly or after addition of a suitable linker and/or a nucleus transfer signal, and the like. As the promoter that is functional in T cells, SR**α** promoter, SV40 promoter, LTR promoter, CMV (cytomegalovirus) promoter, RSV (Rous sarcoma virus) promoter, MoMuLV (Moloney mouse leukemia virus) LTR, HSV-TK (simple herpes virus thymidine kinase) promoter and the like that are constitutional in mammalian cells can be used, but it is not limited to these. In addition, gene promoters such as CD3, CD4, CD8 and the like that are specifically expressed in T cells can also be used.

RNA, preferably mRNA, encoding CAR can be prepared by transcription into mRNA by an in vitro transcription system known per se, using an expression vector containing the aforementioned DNA encoding CAR as a template.

### (b) Nucleic acid encoding exogenous TCR

In the present specification, the "T cell receptor (TCR) " means a receptor that is composed of a dimer of TCR chain (a chain, β chain), recognizes an antigen or the antigen-HLA (human leukocyte type antigen) (MHC; major histocompatibility complex) complex, and transmits stimulation signal to T cells. Each TCR chain is composed of a variable region and a constant region, and the variable region has three complementarity determining regions (CDR1, CDR2, CDR3). The TCR used in the present invention includes not only those in which the α chain and β chain of the TCR form a heterodimer, but also those in which they form a homodimer. Furthermore, the TCR includes those lacking a part or all of the constant region, those having a recombinant amino acid sequence, those having a soluble TCR, and the like.

The "exogenous TCR" means that it is exogenous to T cell which is the target cell in the present invention. The amino acid sequence of the exogenous TCR may be the same as or different from the endogenous TCR expressed by T cell which is the target cell in the present invention.

The nucleic acid encoding the TCR used in the present invention is a nucleic acid encoding α chain and β chain of the TCR that can specifically recognize a surface antigen (e.g., cancer antigen peptide, etc.) to be recognized by the target T cell.

The nucleic acid can be prepared by a method known per se. When the amino acid sequence or nucleic acid sequence of the target TCR is known, for example, a DNA encoding the entire length or part of the TCR of the present invention can be constructed by, based on the sequence, chemically synthesizing DNA chain or RNA chain or connecting synthesized partlyoverlapping short oligo DNA chains by using the PCR method or the Gibson Assembly method.

When the sequence of the target TCR is not known, for example, the target T cell is isolated from the cell population containing the T cell expressing the target TCR, and the nucleic acid encoding the TCR can be obtained from this T cell. Specifically, a cell population containing T cells (e.g., PBMC) is collected from a living body (e.g., human) and cultured under stimulation in the presence of an epitope of a cell surface antigen recognized by the target TCR, and a T cell that specifically recognizes a cell expressing the cell surface antigen can be selected from this cell population by a known method, by using the specificity for the cell expressing the cell surface antigen, and the cell surface antigen such as CD8, CD4, or the like as indices. The specificity of T cell for cells expressing the surface antigen can be measured using, for example, Dextramer assay, ELISPOT assay, cytotoxicity assay, or the like. The aforementioned cell population containing the T cells is preferably collected from, for example, a living body having many cells expressing a cell surface antigen recognized by the target TCR (e.g., patient with disease such as cancer, and the like or T cell-containing population in contact with epitope of the antigen or dendritic cell pulsed with the epitope).

The nucleic acid of the present invention is obtained by extracting DNA from the aforementioned isolated T cells by a conventional method, and amplifying and cloning the TCR gene based on the nucleic acid sequence of the constant region of TCR, by using the DNA as a template. In addition, it can also be prepared by extracting RNA from cells by a conventional method, synthesizing cDNA, and performing 5'-RACE (Rapid amplification of cDNA ends) by using the cDNA as a template, and using an antisense primer complementary to the nucleic acid encoding the constant region of the TCRα chain and β chain. 5'-RACE may be performed by a known method, and can be performed, for example, by using a commercially available kit such as SMART PCR cDNA Synthesis Kit (manufactured by Clontech). The obtained DNA encoding the α chain and β chain of the TCR can be inserted into an appropriate expression vector in the same manner as the aforementioned DNA encoding the CAR. The DNA encoding the α chain and the DNA encoding the β chain may be inserted into the same vector or separate vectors. When they are inserted into the same vector, the expression vector may express both chains polycistronically or monocistronically. In the former case, an intervening sequence that allows polycistronic expression such as IRES or FMV 2A is inserted between the DNAs encoding both chains.

In addition, RNA encoding each chain of TCR, preferably mRNA, can be prepared, for example, using the expression vector as a template and in the same manner as in the aforementioned RNA encoding CAR.

### III. The preparation of the present invention

The present invention also provides a preparation comprising a combination of a nucleic acid or a protein, and ultrafine bubble water or ultrafine bubble aqueous solution comprising ultrafine bubble with an average diameter of not more than 200 nm and not containing phospholipid, for delivering the nucleic acid or protein into an immune cell, wherein an effective amount (amount at which immune cell exerts a desired effect) of the nucleic acid or protein is delivered into the immune cell by the combined use with ultrasound exposure (hereinafter to be also referred to as "the preparation of the present invention"). As used herein, the "immune cell" means the same as that defined for the above-mentioned system of the present invention.

In the preparation of the present invention, the nucleic acid or protein can be the nucleic acid or protein exemplified in the above-mentioned "delivery-increasing method of the present invention". As the ultrafine bubble water or ultrafine bubble aqueous solution in the preparation of the present invention, the ultrafine bubble water or ultrafine bubble aqueous solution described in the above-mentioned "system of the present invention" may be used.

In the formulation of the present invention, a nucleic acid or protein may be mixed with the ultrafine bubble water, etc. in the present invention and administered to the subject as a single preparation, or they may be separately formulated and administered at the same time or at different times by the same or different routes as long as they can simultaneously coexist adjacent to an immune cell.

When the nucleic acid or protein and the ultrafine bubble water, etc. in the present invention are separately formulated, the nucleic acid or protein can be mixed with a pharmaceutically acceptable carrier in an amount that can be tolerated by human or other mammals.

Examples of the pharmaceutically acceptable carrier include pH adjusters such as monosodium phosphate, dipotassium phosphate, disodium phosphate, monopotassium phosphate, sodium hydroxide, hydrochloric acid and the like; antibiotics such as kanamycin sulfate, erythromycin lactobionate, penicillin G potassium and the like; stabilizers such as lactose, potassium glutamate, D-sorbitol, aminoacetic acid, human serum albumin and the like; colorants such as phenol red and the like; isotonicity agents such as sodium chloride, potassium chloride and the like, and the like.

The content of the nucleic acid or protein in the preparation of the present invention is not particularly limited as long as preferable properties such as desired physiological activity and the like can be imparted to the immune cells when they are delivered into the cells by ultrasound exposure. For example, when the preparation of the present invention contains a nucleic acid encoding CAR or exogenous TCR as the nucleic acid and is administered to mammals including humans, the amount of the nucleic acid encoding CAR or exogenous TCR to be administered at one time is in the range of 0.001 mg - 10 mg per 1 kg/body weight. For example, when administered to a human patient, it is administered in the range of 0.001 - 50 mg to a patient weighing 60 kg. The above-mentioned dose is an example, and the dose can be appropriately selected according to the type of nucleic acid to be used, administration route, age, body weight, symptom, and the like of the subject of administration or patient. On the other hand, the amount of ultrafine bubble water, etc. is not particularly limited as long as it is sufficient to deliver a nucleic acid or a protein into an immune cell by ultrasound exposure. For example, an amount permitting the delivery of ultrafine bubbles such that the ultrafine bubble density near the immune cell is 1×10⁸ - 10×10⁸ bubbles/mL, preferably 2×10⁸ - 5×10⁸ bubbles/mL.

### IV. The delivery method of the present invention

The present invention also provides a method for delivering a nucleic acid or a protein into an immune cell, including contacting the cell with the above-mentioned preparation of the present invention, and treating the cell with ultrasound. As used herein, the "immune cell" means the same as that defined for the above-mentioned system of the present invention.

The target to which "the delivery-increasing method of the present invention" or "the delivery method of the present invention" is applied is not particularly limited as long as it is an immune cell collected from the living body (to be also referred to as "ex vivo immune cell" in the present specification) or an immune cell in the tissue or the body of an animal containing same (to be also referred to as "in vivo immune cell" in the present specification). For example, human and other mammals (e.g., mouse, rat, rabbit, dog, cat, bovine, horse, swine, monkey, etc.), an immune cell derived from them or a tissue containing same, and the like can be mentioned. The means for administering the ultrafine bubble water or the like in the present invention to the subject is not particularly limited as long as it is an administration route capable of delivering the ultrafine bubbles to the vicinity of immune cells. In the following, ex vivo immune cells and in vivo immune cells are described separately using, as a specific example, the case of delivering a nucleic acid encoding CAR or exogenous TCR as the nucleic acid.

### (a) Delivery of nucleic acid encoding CAR or exogenous TCR to ex vivo immune cell

According to the delivery method of the present invention, an ex vivo immune cell that expresses CAR or exogenous TCR can be produced by delivering a nucleic acid encoding the CAR or the exogenous TCR into the ex vivo immune cell by combining ultrafine bubble water, etc. and ultrasound in the present invention. Therefore, the present invention also provides an ex vivo immune cell obtained by the method. The "immune cell" here is not particularly limited as long as it is a cell having the ability to damage target cells (pathogenic cells) such as cancer cell and the like by some action mechanism (so-called immune effector cell), among those defined in the above-mentioned system of the present invention. Examples thereof include T cells responsible for cellular immunity among the acquired immunities, NK cells, monocytes, macrophages, dendritic cells, etc. responsible for natural immunity, as well as NKT cells which are T cells having the properties of NK cell, and the like. In a preferred one embodiment, the immune cell can be a T cell. The T cells collected from a living body are also referred to as "ex vivo T cells" in the present specification. On the other hand, in another preferred embodiment, the immune cell can be a cell responsible for natural immunity such as NK cell, macrophage, dendritic cell, and the like. Even when T cells have the same MHC type, there is a considerable risk of causing GVHD by allogeneic (allo) transplantation, whereas alloNK cells and the like are considered to cause no GVHD. Therefore, when various MHC types of allo ex vivo immune cells are prepared, they can be used off-the-self. For CAR-NK cells, for example, CAR-dendritic cells are described in US2016/0096892, Mol Ther. 25(8): 1769-1781 (2017) and the like, and CAR-macrophage and the like are described in, for example, WO 2017/019848, eLIFE. 2018 e36688 and the like.

The ex vivo immune cell into which a nucleic acid encoding CAR or exogenous TCR is introduced by the preparation of the present invention may be an isolated and purified specific immune cell (e.g., T cell, NK cell, monocyte, macrophage, dendritic cell, etc., NKT cell or stem/progenitor cell thereof) or a non-uniform cell population including multiple types of immune cells, such as lymphocytes, as long as it is a cell population containing the above-mentioned effector immune cell, or unipotent or pluripotent stem cells or progenitor cells thereof capable of finally differentiating into them (excluding embryos and other totipotent or pluripotent stem cells). Examples of the isolated and purified T cell include cytotoxic T cell (CTL) which is a CD8-positive cell, helper T cell, regulatory T cell, effector T cell, and the like which are CD4-positive cells, and cytotoxic T cell is preferred.

The aforementioned lymphocyte can be collected from, for example, peripheral blood, bone marrow and cord blood of human or non-human mammals, or produced from stem cells such as iPS cell and the like. When the ex vivo immune cell (e.g., ex vivo T cell) into which a nucleic acid encoding CAR or exogenous TCR has been introduced by the preparation of the present invention is used for the treatment of diseases such as cancer and the like, the cell population is preferably collected from a subject to be treated itself or a donor matched to the MHC type of the subject to be treated.

Examples of the unipotent or pluripotent stem cells or progenitor cells thereof capable of finally differentiating into immune cell include hematopoietic stem cell, myelolymphoid progenitor (MLP) cell, myeloid progenitor (MP) cell, granulo-monocyte progenitor (GMP) cell, macrophage-dendritic cell progenitor (MDP) cell, dendritic cell precursor (DCP) cell, and the like. These stem/progenitor cells can be differentiated into various immune cells, for example, E cells by a method known per se.

The method for contacting the preparation of the present invention with ex vivo immune cells is not particularly limited and, for example, the preparation of the present invention may be added to a general medium for immune cells. Since immune cells proliferate in a floating state without adhering to a culture container, preculture of ex vivo immune cells and the contact with the preparation of the present invention are preferably performed in a culture container coated with a non-adhesive or low-adhesive substrate. Ultrasound exposure can be performed on ex vivo immune cells by using the ultrasound generator and irradiation conditions described with regard to the system of the present invention.

When the preparation of the present invention particularly contains a nucleic acid encoding exogenous TCR as an active ingredient, the expression of the endogenous TCRα chain and TCRβ chain originally expressed by the T cell may be suppressed by siRNA from the aspects of an increase in the expression of exogenous TCR, suppression of the appearance of mispair TCR, or suppression of self-reactivity. When the aforementioned nucleic acid is applied to the method, in order to avoid the effect of siRNA on exogenous TCR, it is preferable that the base sequence of the nucleic acid encoding the TCR is different from the base sequence corresponding to RNA on which siRNA that suppresses the expression of endogenous TCRα chain and TCRβ chain acts (codon conversion type sequence). These methods are described in, for example, WO 2008/153029. The aforementioned base sequence can be produced by introducing a silent mutation into a nucleic acid encoding TCR obtained from nature, or by chemically synthesizing an artificially designed nucleic acid. Alternatively, to avoid mispairing with endogenous TCR chains, a part or all of the constant regions of the nucleic acid encoding the exogenous TCR may be replaced with constant regions derived from an animal other than human, for example, mouse.

### (b) Delivery of nucleic acid encoding CAR or exogenous TCR to in vivo immune cell

According to the delivery method of the present invention, the preparation of the present invention is administered to mammals (human or other mammal (e.g., mouse, rat, hamster, rabbit, cat, dog, bovine, sheep, monkey), preferably human) and tissue or organ containing immune cells(e.g., spleen, thymus, etc.) is exposed to ultrasound, whereby the nucleic acid is introduced into immune cells, for example, T cells (to be also referred to as "in vivo T cells" in the present specification) in the animal body, whereby the expression of CAR or exogenous TCR can be induced. The in vivo immune cells specifically recognize cancer cells, etc. that express a surface antigen targeted by CAR or exogenous TCR, and kill the diseased cells, whereby the prophylactic or therapeutic effects on the disease can be shown.

The preparation of the present invention in the form of, for example, injection can deliver the nucleic acid and ultrafine bubble water, etc. to the vicinity of in vivo immune cells by subcutaneous injection, intravenous injection, intramuscular injection, intraperitoneal injection, drip injection, intracerebral injection, intra-cerebrospinal fluid injection or the like. In addition, the ultrasound exposure can be performed by an operation conventionally used for ultrasound diagnosis, by substituting the target region with a tissue or organ containing in vivo immune cell.

When "the delivery-increasing method of the present invention" or "the delivery method of the present invention" is applied to an ex vivo immune cell, that is, an immune cell isolated from an animal, or a stem cell such as iPS cell and the like, using a method known per se, a step of culturing the immune cell after a transfection treatment may be included. The culturing step can be performed using a culture container (e.g., dish, flask, etc.) and a culture apparatus (e.g., CO₂ incubator, etc.) known per se, in a medium generally used for maintenance culture of terminally differentiated immune cells or inducing differentiation of more undifferentiated stem/progenitor cell into terminally differentiated immune cell. Since immune cells proliferate in a floating state without adhering to a culture container, culture of ex vivo immune cells is preferably performed in a culture container coated with a non-adhesive or low-adhesive substrate. In addition, the above-mentioned culture step also includes a passage culture step. When cells are passage cultured by "the delivery-increasing method of the present invention" or "the delivery method of the present invention", the expression intensity of the target substance introduced into the cell can be maintained.

### V. Medicament containing ex vivo immune cell into which target substance has been introduced by the present invention

The ex vivo immune cell into which the target substance has been introduced using the system of the present invention or by the delivery method of the present invention provides desired effects (e.g., acquisition of new physiological activity) by the action of the target substance. Therefore, it can be directly formulated as a pharmaceutical composition, or by mixing with known pharmaceutically acceptable carriers (including excipient, diluent, filler, binder, lubricant, flow aid, disintegrant, surfactant, etc., and the like), conventionally-used additives, and the like. Excipients are well known to those of skill in the art, and, adjuvants such as wetting agent or emulsifier and the like, and pH buffering agents can also be used. Furthermore, formulation adjuvants such as suspending agent, preservative, stabilizer, dispersing agent, and the like, and the like may also be used. The above-mentioned pharmaceutical composition may be in a dry form to be reconstituted with a suitable sterile liquid before use. The pharmaceutical composition can be systemically or locally administered orally or parenterally depending on the form of preparation (oral agents such as tablet, pill, capsule, powder, granule, syrup, emulsion, suspension and the like; parenteral agents such as injection, drip transfusion, external preparation, suppository and the like) and the like. In the case of parenteral administration, intravenous administration, intradermal administration, subcutaneous administration, rectal administration, transdermal administration and the like are possible. In addition, when used in the injection form, an acceptable buffering agent, solubilizing agent, isotonic agent, and the like can also be added.

For example, when the target substance is a nucleic acid encoding CAR or exogenous TCR, the ex vivo immune cells into which the target substance has been introduced can specifically recognize cells expressing a surface antigen specifically recognized by the CAR or exogenous TCR and kill them (e.g., inducing apoptosis). Therefore, when a nucleic acid encoding CAR or exogenous TCR that recognizes a surface molecule that is specifically expressed or whose expression is enhanced in a diseased cell such as cancer cell or the like is contained as an active ingredient, an ex vivo immune cell into which the nucleic acid has been introduced and expressing the CAR or exogenous TCR can be used for the prophylaxis or treatment of diseases such as cancer, and can be safely administered to humans or other mammals (e.g., mouse, rat, hamster, rabbit, cat, dog, bovine, sheep, monkey), preferably human.

In a preferred embodiment, a medicament containing an ex vivo immune cell into which the target substance has been introduced by the present invention can be a prophylactic or therapeutic drug for cancer. The cancer to be the application target for the medicament is not particularly limited. Examples thereof include, but are not limited to, acute lymphocytic cancer, alveolar rhabdomyosarcoma, bladder cancer, bone cancer, brain cancer (e.g., medulloblastoma), breast cancer, anus, anal canal or anus rectal cancer, eye cancer, intrahepatic bile duct cancer, articular cancer, neck, gall bladder or pleural cancer, nasal, nasal cavity or middle ear cancer, oral cancer, vulvar cancer, chronic myelogenous cancer, colorectal cancer, esophageal cancer, cervical cancer, fibrosarcoma, gastrointestinal carcinoid tumor, head and neck cancer (e.g., head and neck squamous cell carcinoma), hypopharyngeal cancer, kidney cancer, laryngeal cancer, leukemia (e.g., acute lymphoblastic leukemia, acute lymphocytic leukemia, chronic lymphocytic leukemia, acute myelogenous leukemia), liquid tumor, liver cancer, lung cancer (e.g., non-small cell lung cancer), lymphoma (e.g., Hodgkin lymphoma, non-Hodgkin's lymphoma, diffuse large B cell lymphoma, follicular lymphoma), malignant mesothelioma, mastocytoma, melanoma, multiple myeloma, nasopharynx cancer, ovarian cancer, pancreatic cancer; peritoneal, retinal and mesenteric cancer; pharyngeal cancer, prostate cancer, rectal cancer, kidney cancer, skin cancer, small intestinal cancer, soft tissue cancer, solid tumor, gastric cancer, testicular tumor, thyroid cancer, ureteral cancer and the like.

As the dose of the medicament containing an ex vivo immune cell into which the target substance has been introduced by the present invention, for example, the nucleic acid encoding CAR or exogenous TCR in an amount in the range of 0.001 mg - 10 mg per 1 kg/body weight is administered at one time. For example, for administration to a human patient, 0.001 - 50 mg is administered to a patient weighing 60 kg. The above-mentioned dose is an example, and the dose can be appropriately selected according to the type of nucleic acid to be used, administration route, age, body weight, symptom, and the like of the subject of administration or patient.

The medicament containing an ex vivo immune cell into which the target substance has been introduced by the present invention is preferably administered parenterally to a subject. The parenteral administration method includes intravenous, intraarterial, intramuscular, intraperitoneal, and subcutaneous administration methods, and the like. The dose is appropriately selected according to the condition, body weight, age, etc. of the subject. Generally, it is administered such that the cell number is generally 1×10⁶ - 1×10¹⁰ cells, preferably 1×10⁷ - 1×10⁹ cells, more preferably 5×10⁷ - 5×10⁸ cells, per administration to a subject with a body weight of 60 kg. In addition, it may be administered once or administered in multiple portions.

The present invention is further described in the following by Reference Examples and Examples; however, the present invention is not limited to them in any sense.

In the following Reference Examples and Examples, "%" indicates weight/volume % unless otherwise specified.

### [Example]

### Reference Example 1: Preparation of ultrafine bubble water

Polysorbate 80 (2 g) (0.1% polysorbate 80) was dissolved in water for injection (2L) or diluted Mclivaine buffer (pH3.0, 2 L), and ultrafine bubble aqueous solution was prepared using an ultrafine bubble generator (nanoGALF^{™} FZ1N-02) manufactured by IDEC with the following settings. Positively-charged ultrafine bubble can be produced when an acidic buffer (diluted Mclivaine buffer: pH 3.0) is used, and negatively-charged ultrafine bubble can be produced when water for injection is used.
gas used for preparation: air
bubble water flow: about 4.0 L/min
dissolution pressure: 300 KPa ± 5% (s.d.)

The prepared ultrafine bubble aqueous solution was subjected to high-pressure vapor sterilization using an autoclave as appropriate at 121 - 124°C for 30 min. After sterilization, ultrafine bubble average diameter, ultrafine bubble density and d90/d10 ratio were measured by a tracking method utilizing laser beam scattering using LM10, NanoSight Ltd.

The results are shown below.
ultrafine bubble average diameter: 120 nm ± 16 nm
ultrafine bubble density: 4×10⁸ bubbles/mL
d90/d10 ratio: 3.3

### Example 1 Introduction of gene into T cell

Jurkat E6.1 (3×10⁴ cells, 0.5 mL) in 10% FBS-containing RPMI 1640 medium was seeded in 48 wells. After removing the medium, 0.5 mL of ultrafine bubble/RPMI medium containing 5 µg of pGFP was added. An ultrafine bubble/RPMI medium prepared by adding 1 L of the ultrafine bubble aqueous solution prepared in Reference Example 1 to a RPMI medium powder, and adding 2 g/L NaHCO₃ and 10% FBS was used as a transfection medium.

After adding the pGFP-containing medium, ultrasound exposure was performed for 10 sec at a frequency of 1 MHz and an output intensity of 0.5 W/cm² using an ultrasound generator (NEPAGENE). The cells were cultured for 2 hr, then the transfection medium was removed, and the cells were cultured in RPMI medium (culture medium) for 48 hr. Thereafter, the cells were collected, completely lysed with 0.15 mL of cell lysis solution, and the fluorescent intensity of GFP was measured with a spectrofluoro-photometer. The number of surviving cells was also measured, converted into the fluorescent intensity per surviving cell, and compared.

The results are shown in Fig. 1.

### Example 2 Introduction of protein into T cell

Jurkat E6.1 (3×10⁴ cells, 0.5 mL) in 10% FBS-containing RPMI 1640 medium was seeded in 48 wells. After removing the medium, 0.5 mL of ultrafine bubble/RPMI medium containing 5 µg of IgG-FITC (4 mL of ultrafine bubble/RPMI medium was added to 40 µL of IgG-FITC) was added. An ultrafine bubble/RPMI medium prepared by adding 1 L of the ultrafine bubble aqueous solution prepared in Reference Example 1 to a RPMI medium powder, and adding 2 g/L NaHCO₃ and 10% FBS was used as a transfection medium.

After adding the IgG-FITC-containing medium, ultrasound exposure was performed for 10 sec at a frequency of 1 MHz and an output intensity of 0.5 W/cm² using an ultrasound generator (NEPAGENE). The cells were cultured for 2 hr, then the transfection medium was removed, and the cells were cultured in RPMI medium (culture medium) for 48 hr. The fluorescent intensity of IgG-FITC was measured with a spectrofluoro-photometer. The number of surviving cells was also measured, converted into the fluorescent intensity per surviving cell, and compared.

The results are shown in Fig. 2.

### [Industrial Applicability]

The system of the present invention can efficiently deliver a target substance such as nucleic acid, protein, or the like into an immune cell by ultrasound exposure at a low output intensity, and thus can provide a highly safe system for drug delivery into an immune cell. As compared with the use of conventional microbubbles, the introduction efficiency of a target substance into a cell can be remarkably increased using the ultrafine bubbles. Furthermore, since the system does not require use of liposome, it can be manufactured at a low cost and is highly safe. From the above, the system of the present invention is extremely useful as a novel DDS into an immune cell.

This application is based on a patent application No. 2019-119164 filed in Japan (filing date: June 26, 2019), the contents of which are incorporated in full herein.

## Claims

1. A system for delivering a target substance into an immune cell, comprising ultrafine bubble water or ultrafine bubble aqueous solution comprising ultrafine bubbles with an average diameter of not more than 200 nm and not containing phospholipid, and an ultrasound generator in combination.

2. The system according to claim 1, wherein the ultrafine bubble aqueous solution comprises one or more components selected from one or more kinds of surfactants selected from an anionic surfactant, a non-ionic surfactant, a cationic surfactant and an amphoteric surfactant, a hydrophilic resin, and a buffer.

3. The system according to claim 1, wherein the ultrafine bubble aqueous solution consists of a non-ionic surfactant and/or a hydrophilic resin.

4. The system according to claim 1, wherein the ultrafine bubble is constituted of perfluorohydrocarbon or air.

5. The system according to claim 1, wherein the ultrafine bubble has an average diameter of 50 nm - 200 nm.

6. The system according to claim 1, wherein the ultrafine bubble has a d90/d10 ratio of not more than 5.

7. The system according to claim 1, wherein the ultrafine bubble in the ultrafine bubble water or ultrafine bubble aqueous solution has a density of not less than 1.0×10⁸ bubbles/mL.

8. The system according to claim 1, wherein ultrasound output intensity in the ultrasound generator is not more than 720 mW/cm².

9. The system according to claim 1, wherein in the ultrasound generator, ultrasound output intensity is 50 - 500 mW/cm² and ultrasound frequency is 0.5 - 10 MHz.

10. The system according to claim 1, wherein the target substance is a nucleic acid or a protein.

11. The system according to claim 10, wherein the nucleic acid encodes a chimeric antigen receptor or an exogenous T cell receptor.

12. The system according to claim 1, wherein the immune cell is T cell.

13. The system according to claim 11, for delivering a nucleic acid into an immune cell.

14. A method for increasing the delivery of a nucleic acid or a protein into an immune cell by using ultrafine bubble water or ultrafine bubble aqueous solution comprising ultrafine bubble with an average diameter of not more than 200 nm and not containing phospholipid, and ultrasound.

15. A preparation comprising a combination of a nucleic acid or a protein, and ultrafine bubble water or ultrafine bubble aqueous solution comprising ultrafine bubble with an average diameter of not more than 200 nm and not containing phospholipid, for delivering the nucleic acid or protein into an immune cell, wherein an effective amount of the nucleic acid or protein is delivered into the immune cell by the combined use with ultrasound exposure.

16. A method for delivering a nucleic acid or a protein into an immune cell by contacting the cell with the preparation according to claim 15, and treating them with ultrasound.
